# EUROPEAN PATENT APPLICATION

(11) **EP 1 424 324 A1**
(43) Date of publication of application: **02.06.2004**
(21) Application number: 02406037.8
(22) Date of filing: 28.11.2002
(51) Int. Cl.: C07D 207/34, A61K 31/40

(54) **Crystalline form F of Atorvastatin hemi-calcium salt**

(71) Applicant: Teva Pharmaceutical Industries Limited, 49131 Petah Tikvah (IL)
(72) Inventor: Blatter, Fritz, 4153 Reinach (CH); Szelagiewicz, Martin, 4142 Munchenstein (CH); Van der Schaaf, Paul Adriaan, 4123 Allschwil (CH)
(74) Representative: Nachshen, Neil Jacob

(57) **Abstract**

The present invention is directed to the novel polymorphic Form F of Atorvastatin calcium, processes for the preparation therof and pharmaceutical compositions comprising this crystalline form.

## Description

The present invention is directed to a crystalline form of Atorvastatin calcium, processes for the preparation thereof and pharmaceutical compositions comprising this crystalline form.

The present invention relates to a crystalline form of Atorvastatin calcium. Atorvastatin calcium is known by the chemical name, [R-(R*,R*)]-2-(4-fluoropheny))-beta,delta-dihydroxy-5-(1-methylethy))-3-phenyl-4-[(phenylamino)carbonyl]-1H-pyrrole-1-heptanoic acid calcium salt (2:1). Atorvastatin has the following formula:

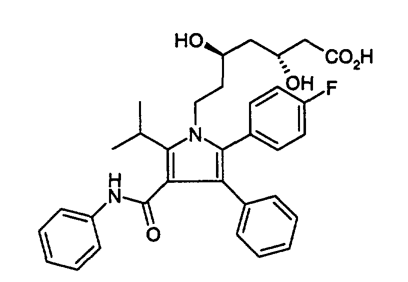

Atorvastatin calcium is an orally-active hypocholesterolaemic, a liver-selective HMG-CoA reductase inhibitor. Processes for the preparation of Atorvastatin calcium are described in US-A-5,273,995, US-A-5,298,627, US-A-6,087,511, US-A-6,274,740, WO-A-97/03960, WO-A-02/059087, WO-A-02/072073, and in the publications by P.L. Brower et al. in Tetrahedron Letters (1992), vol. 33, pages 2279-2282, K.L. Baumann et al. in Tetrahedron Letters (1992), vol. 33, pages 2283-2284 and A. Graul et al. in Drugs of the Future (1997), vol. 22, pages 956-968.

This calcium salt (2:1) is desirable since it enables Atorvastatin calcium to be conveniently formulated. The processes in the above mentioned patents and publications result in the preparation of amorphous Atorvastatin calcium.

The preparations of Atorvastatin calcium (2:1) described in WO-A-97/03958 and WO-A-97/03959 result in the isolation of crystalline Atorvastatin calcium with the polymorphic forms III, and I, II, and IV, respectively. WO-A-01/36384, WO-A-02/41834 and WO-A-02/43732 claim the preparation of crystalline Atorvastatin calcium with the polymorphic forms V to XII, whereas WO-A-02/051804 claims the polymorphic forms A, B1, B2, C, D and E. However, there is still a need to produce Atorvastatin calcium in a reproducible, pure and crystalline form to enable formulations to meet exacting pharmaceutical requirements and specifications. Furthermore, it is economically desirable that the product is stable for extended periods of time without the need for specialised storage conditions.

Surprisingly, there has now been found a novel crystalline form of Atorvastatin calcium salt (2:1), herein designated as Form F. This novel form of the present invention can be prepared in ecological friendly solvents and has a good thermal stability combined with good solubility characterisitics.

Accordingly, the present invention is directed to the polymorphic Form F of Atorvastatin calcium salt (2:1).

Therefore, the present invention is directed to a crystalline polymorph F of [R-(R*,R*)]-2-(4-fluorophenyl)-beta,delta-dihydroxy-5-(1-methylethyl)-3-phenyl-4-[(phenylamino)carbonyl]-1 H-pyrrole-1-heptanoic acid calcium salt (2:1) which exhibits a characteristic X-ray powder diffraction pattern with characteristic peaks expressed in d-values (Å) at 24.3 (s), 10.2 (s), 8.6 (s), 4.57 (vs), 4.26 (m); wherein (vs) = very strong intensity; (s) = strong intensity; (m) = medium intensity.

More particularly, the crystalline polymorph F exhibits a characteristic X-ray powder diffraction pattern with characteristic peaks expressed in d-values (Å) at 32.3 (w), 24.3 (s), 16.5 (m), 13.0 (w), 11.4 (m), 10.2 (s), 8.6 (s), 7.0 (m), 6.4 (m), 5.16 (m), 4.96 (m), 4.57 (vs), 4.26 (m), 3.95 (m), 3.67 (m), 3.48 (m), 3.20 (w). The abbreviations in brackets mean: (vs) = very strong intensity; (s) = strong intensity; (m) = medium intensity; (w) = weak intensity.

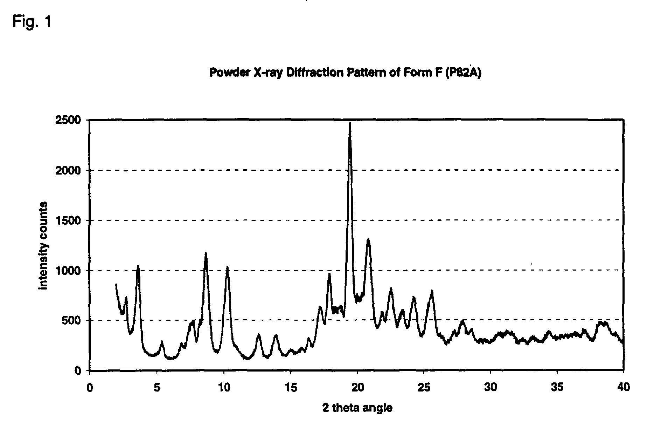

The polymorphic form F of Atorvastatin calcium is especially characterized by a powder X-ray diffraction pattern substantially as depicted in Figure 1.

The powder X-ray diffraction pattern of form F may appear with a small shoulder on the left side of the peak at 2θ = 7.3° (d = 11.4 Å). Similarly, a small shoulder may appear on the left side of the peak at 2θ = 8.6° (d = 10.2 Å), see Figure 1. Two additional peaks at d = 4.85 Å and d = 4.75 Å exhibit slightly variable intensities.

Furthermore, the present invention is directed to processes for the preparation of Form F of Atorvastatin calcium.

Form F can generally be prepared by adding Form A to a ketone solvent, especially acetone. It is preferred that the ketone solvent contains as a further solvent some water. The amount of water is preferably about 1 to 30%, more preferably about 5 to 20%, especially about 10 to 20% by volume of the suspension. It is preferred that the suspension is treated at temperatures between 10 and 60°C, preferably at temperatures of 20 to 40°C, especially for a longer periods of time, like 10 to 40 hours. If desired, during the preparation process seeding with Form F can be carried out. Form F can, for example, be isolated by filtration and dried in air or in vacuum. The above mentioned process can also be carried out using another crystalline form or the amorphous form of atorvastatin calcium. Examples of other crystalline forms are Forms I, II, III, IV, V, VI, VII, VIII, IX, X, XI, XII, A, B1, B2, C, D and E, which are disclosed and characterised in the references given hereinbefore. Preferred forms for this purpose are Form A (see for example WO-A-02/051804; last but one paragraph of page 2; page 4, last but one paragraph to page 5, first paragraph; Examples 2, 8 and 9; Fig. 2) or Form I (see for example WO-A-97/03959; table on page 4; page 20, line 9 to page 22, line 11; Example 1; Fig. 1).

Form F can also be prepared from Atorvastatin lactone upon subsequent reaction with NaOH to form Atorvastatin sodium followed by reaction with CaCl₂ in a ketone solvent, especially in acetone. It is preferred that the ketone solvent contains as a further solvent some water. The amount of water is preferably about 1 to 30%. If desired, during the preparation process seeding with Form F can be carried out.

Form F can also be prepared directly from Atorvastatin lactone upon reaction with a calcium(II) salt, like Ca(OH)₂ or Ca(OAc)₂, in a ketone solvent, especially in acetone. It is preferred that the ketone solution contains as a further solvent some water. The amount of water is preferably about 1 to 30%. If desired, during the preparation process seeding with Form F can be carried out.

Form F can also be prepared by adding a concentrated solution of Atorvastatin calcium in an organic solvent, like tetrahydrofuran, to a ketone solvent, especially acetone. It is preferred that the ketone solution contains as a further solvent some water. The amount of water is preferably about 1 to 30%. If desired, during the preparation process seeding with Form F can be carried out.

As to the ketone solvent of the preparation processes given above it is preferred to use C₃-C₈ketones, especially aceton.

Another object of the present invention are pharmaceutical compositions comprising an effective amount of crystalline polymorphic Form F, and a pharmaceutically acceptable carrier.

The polymorphic Form F may be used as single component or as mixtures with other polymorphic forms or the amorphous form of atorvastatin calcium.

As to Atorvastatin calcium it is preferred that it contains 25-100% by weight, especially 50-100% by weight of the novel form, based on the total amount of Atorvastatin calcium. Preferably, such an amount of the novel polymorphic form of Atorvastatin calcium is 75-100% by weight, especially 90-100% by weight. Highly preferred is an amount of 95-100% by weight.

The compositions of the present invention include powders, granulates, aggregates and other solid compositions comprising polymorphic Form F. In addition, the compositions that are contemplated by the present invention may further include diluents, such as cellulose-derived materials like powdered cellulose, microcrystalline cellulose, microfine cellulose, methyl cellulose, ethyl cellulose, hydroxyethyl cellulose, hydroxypropyl cellulose, hydroxypropylmethyl cellulose, carboxymethyl cellulose salts and other substituted and unsubstituted celluloses; starch; pregelatinized starch; inorganic diluents like calcium carbonate and calcium diphosphate and other diluents known to the pharmaceutical industry. Yet other suitable diluents include waxes, sugars and sugar alcohols like mannitol and sorbitol, acrylate polymers and copolymers, as well as pectin, dextrin and gelatin.

Further excipients that are within the contemplation of the present invention include binders, such as acacia gum, pregelatinized starch, sodium alginate, glucose and other binders used in wet and dry granulation and direct compression tableting processes. Excipients that also may be present in the solid compositions further include disintegrants like sodium starch glycolate, crospovidone, low-substituted hydroxypropyl cellulose and others. In addition, excipients may include tableting lubricants like magnesium and calcium stearate and sodium stearyl fumarate; flavorings; sweeteners; preservatives; pharmaceutically acceptable dyes and glidants such as silicon dioxide.

The dosages include dosages suitable for oral, buccal, rectal, parenteral (including subcutaneous, intramuscular, and intravenous), inhalant and ophthalmic administration. Although the most suitable route in any given case will depend on the nature and severity of the condition being treated, the most preferred route of the present invention is oral. The dosages may be conveniently presented in unit dosage form and prepared by any of the methods well-known in the art of pharmacy.

Dosage forms include solid dosage forms, like tablets, powders, capsules, suppositories, sachets, troches and losenges as well as liquid suspensions and elixirs. While the description is not intended to be limiting, the invention is also not intended to pertain to true solutions of Atorvastatin calcium whereupon the properties that distinguish the solid form of Atorvastatin calcium are lost. However, the use of the novel form to prepare such solutions is considered to be within the contemplation of the invention.

Capsule dosages, of course, will contain the solid composition within a capsule which may be made of gelatin or other conventional encapsulating material. Tablets and powders may be coated. Tablets and powders may be coated with an enteric coating. The enteric coated powder forms may have coatings comprising phthalic acid cellulose acetate, hydroxypropylmethyl-cellulose phthalate, polyvinyl alcohol phthalate, carboxymethylethylcellulose, a copolymer of styrene and maleic acid, a copolymer of methacrylic acid and methyl methacrylate, and like materials, and if desired, they may be employed with suitable plasticizers and/or extending agents. A coated tablet may have a coating on the surface of the tablet or may be a tablet comprising a powder or granules with an enteric coating.

Preferred unit dosages of the pharmaceutical compositions of this invention typically contain from 1 to 100 mg of the novel Atorvastatin calcium form or mixtures with other forms of Atorvastatin calcium (including the amorphous form). More usually, the combined weight of the Atorvastatin calcium forms of a unit dosage are from 5 mg to 80 mg, for example 10, 20 or 40 mg.

The following Examples illustrate the invention in more detail. Temperatures are given in degrees Celsius.

### Example 1: Preparation of polymorphic Form F

277 mg of Atorvastatin calcium Form A are added to 11 ml of a mixture of acetone and water (80:20 v/v). This suspension is stirred at ambient temperature for about ten minutes, leading to almost complete dissolution of Form A. When the resulting slightly turbid, opalescent solution is stirred at 40°C for about 14 hours, a white precipitate is formed. This precipitate is separated by filtration and dried at 60°C for 2 hours. Yield: 153 mg (55%). Analysis by powder X-ray diffraction shows that the obtained sample is Atorvastatin calcium Form F as shown in Figure 1. Karl Fischer titration of the sample after X-ray diffraction reveals a water content of 2.0 %.

### Example 2: Preparation of polymorphic Form F

303 mg of Atorvastatin calcium Form A are added to a mixture of 10 ml acetone and 1 ml of water. This mixture is stirred at ambient temperature for about 15 minutes which leads to almost complete dissolution of the solid. The slightly turbid, opalescent solution/suspension is stirred at 40°C for 22 hours. Within this time a thick precipitate is formed. This suspension is thoroughly stirred at 50°C for about 15 minutes, then the mixture is cooled to 20°C while stirring is continued for another 4 hours. Then the suspension is filtrated and dried at 80°C for 3 hours (300 mbar). An X-ray powder diffraction study shows the product to be polymorphic Form F.

### Example 3: Preparation of polymorphic Form F

500 mg of Atorvastatin calcium Form I are suspended in 15 ml of aceton and water mixture (80:20 v/v). This suspension is shortly stirred at 60°C giving a clear solution which becomes immediately turbid. This turbid suspension is stirred for an additional 16 hours at 40°C. The resulting precipitate is filtered, washed with 2 ml of the aceton/water mixture and dried for 1 hour at 50°C/800 mbar. Yield 400 mg (80%). An X-ray powder diffraction study shows the product to be polymorphic Form F.

X-rax powder diffraction measurements are performed on a Philips 1710 powder X-ray diffractometer using Cu Kα radiation (Cu Kα₁, and Cu Kα₂ at a ratio of 2, λ of Cu K_{α1} = 1.54060, and λ of Cu Kα₂ = 1.54447). The X-ray source is operated at 45 kV and 45 mA. Spectra are recorded at a step size of 0.02° with a counting time of 2.4 seconds per step. The accuracy of the 2 theta values of conventionally recorded powder X-ray diffraction patterns is generally +/- 0.2°. For sample preparation, about 80 mg of substance are prepared into circular shaped quartz sample holders of 0.5 mm depth and 10 mm width.

### Brief description of the drawing

Figure 1 is a characteristic X-ray powder diffraction pattern for Form F.

## Claims

1. A crystalline polymorph F of [R-(R*,R*)]-2-(4-fluorophenyl)-beta,delta-dihydroxy-5-(1-methylethyl)-3-phenyl-4-[(phenylamino)carbonyl]-1H-pyrrole-1-heptanoic acid calcium salt (2:1) which exhibits a characteristic X-ray powder diffraction pattern with characteristic peaks expressed in d-values (Å) at 24.3 (s), 10.2 (s), 8.6 (s), 4.57 (vs), 4.26 (m); wherein (vs) = very strong intensity; (s) = strong intensity; (m) = medium intensity.

2. A crystalline polymorph F of [R-(R*,R*)]-2-(4-fluorophenyl)-beta,delta-dihydroxy-5-(1-methylethyl)-3-phenyl-4-[(phenylamino)carbonyl]-1H-pyrrole-1-heptanoic acid calcium salt (2:1) which exhibits a characteristic X-ray powder diffraction pattern with characteristic peaks expressed in d-values (Å) at 32.3 (w), 24.3 (s), 16.5 (m), 13.0 (w), 11.4 (m), 10.2 (s), 8.6 (s), 7.0 (m), 6.4 (m), 5.16 (m), 4.96 (m), 4.57 (vs), 4.26 (m), 3.95 (m), 3.67 (m), 3.48 (m), 3.20 (w); wherein (vs) = very strong intensity; (s) = strong intensity; (m) = medium intensity; (w) = weak intensity.

3. A crystalline polymorph F of [R-(R*,R*)]-2-(4-fluorophenyl)-beta,delta-dihydroxy-5-(1-methylethyl)-3-phenyl-4-[(phenylamino)carbonyl]-1 H-pyrrole-1-heptanoic acid calcium salt (2:1) having an X-ray powder diffraction pattern substantially as depicted in figure 1.

4. A process for the preparation of a crystalline polymorph according to any of claims 1 to 3, wherein Atorvastatin calcium is added to a ketone solvent at temperatures between 10 and 60°C.

5. A process according to claim 4 in which Atorvastatin calcium Form A is used.

6. A process according to claim 4 in which the ketone is acetone.

7. A process according to claim 4 in which the ketone contains 1 to 30% water.

8. A process according to any of claims 4 to 7, wherein seeding is carried out with crystals of the crystalline polymorph according to any of claims 1 to 3.

9. A process for the preparation of a crystalline polymorph according to any of claims 1 to 3, wherein Atorvastatin lactone in a ketone solvent is subsequently reacted with NaOH to form Atorvastatin sodium and then with CaCl₂.

10. A process according to claim 9 in which the ketone is acetone.

11. A process according to claim 9 in which the ketone contains 1 to 30% water.

12. A process according to any of claims 9 to 11, wherein seeding is carried out with crystals of the crystalline polymorph according to any of claims 1 to 3.

13. A process for the preparation of a crystalline polymorph according to any of claims 1 to 3, wherein Atorvastatin lactone in a ketone solvent is reacted with a calcium(II) salt, preferably Ca(OH)₂ or Ca(OAc)₂.

14. A process according to claim 13 in which the ketone is acetone.

15. A process according to claim 13 in which the ketone contains 1 to 30% water.

16. A process according to any of claims 13 to 15, wherein seeding is carried out with crystals of the crystalline polymorph according to any of claims 1 to 3.

17. A process for the preparation of a crystalline polymorph according to any of claims 1 to 3, wherein a concentrated solution of Atorvastatin calcium in an organic solvent is added to a ketone solvent.

18. A process according to claim 17 in which the ketone is acetone, and the organic solvent is tetrahydrofuran.

19. A process according to claim 17 in which the ketone contains 1 to 30% water.

20. A process according to any of claims 17 to 19, wherein seeding is carried out with crystals of the crystalline polymorph according to any of claims 1 to 3.

21. A pharmaceutical composition comprising an effective amount of a crystalline polymorphic form according to any of claims 1 to 3, and a pharmaceutically acceptable carrier.
